# EUROPEAN PATENT APPLICATION

(11) **EP 4 541 411 A1**
(43) Date of publication of application: **23.04.2025**
(21) Application number: 24209246.8
(22) Date of filing: 27.08.2019
(51) Int. Cl.: A61M 60/538

(54) **METHOD OF STARTING A BLOOD PUMP WITH AN ELECTRICAL FAULT**

(30) Priority: 19.09.2018 US 201862733338 P
(62) Divisional of application: 19765888.3
(71) Applicant: HeartWare, Inc., Miami Lakes, FL 33014 (US)
(72) Inventor: REYES, Carlos, Davie (US); WOLMAN, Justin, Aventura (US); EGLER, Mark Steven, Reading (US)
(74) Representative: Peterreins Schley

(57) **Abstract**

A control circuit for an implantable blood pump is provided. The blood pump has a first stator and a second stator. The control circuit has processing circuity including one or more processors. The one or more processors are configured to detect an electrical fault in one of a group consisting of the first stator and the second stator; and commence a startup of the first stator and the second stator in response to the electrical fault.

## Description

### TECHNICAL FIELD

The present invention relates to a method and system for starting a blood pump.

### BACKGROUND

Implantable blood pumps used as mechanical circulatory support devices include a pumping mechanism to move blood from the heart to the rest of the body. The pumping mechanism may be a centrifugal flow pump, such as the HVAD^{®} Pump manufactured by HeartWare, Inc. in Miami Lakes, Fla., USA. The HVAD^{®} Pump is further discussed in U.S. Patent No. 8,512,013, the disclosure of which is hereby incorporated herein in the entirety. In operation, the blood pump draws blood from a source, such as the right ventricle, left ventricle, right atrium, or left atrium of a patient's heart and impels the blood into an artery, such as the patient's ascending aorta or peripheral artery.

Known blood pumps, such as the HVAD^{®} pump, typically include an impeller positioned within a pump housing to impel blood through the housing from an inflow end to an outflow end. The HVAD^{®} pump includes dual stators, one located upstream of the impeller and one located downstream from the impeller, configured to rotate the impeller to impel the blood. A control system typically controls operation of a power source or power supply in communication with the stators to drive the impeller at a set rotational speed and thus provide a constant pumping action.

When the blood pump is off, and/or operation of the impeller is temporarily suspended, it is desirable to start or resume operation of the pump as quickly and efficiently as possible to provide assistance to a patient's heart. However, starting the impeller may be difficult, particularly when the impeller is at rest against the housing of the pump. At least one known method of starting a blood pump includes a ramp phase, a commutation phase, and a speed phase in which a pump speed is locked for a time period followed by a speed increase. Another known method of starting a blood pump includes the use of only a single stator when a fault is detected in a second stator which increases the difficulty of a successful startup.

### SUMMARY

The present invention advantageously provides a method and system for starting a blood pump.

In one aspect, the present disclosure provides a method of starting an implantable blood pump. The method includes gradually increasing a motor speed from an inactive state to a first speed during a first- time period. A predetermined maximum voltage is applied during the gradual increase in the motor speed. The gradual increase in motor speed is transitioned to a closed loop speed control to achieve an operating speed, the operating speed being greater than the first speed.

In another aspect, the method further includes bypassing a commutation phase.

In another aspect, the method further includes maintaining a voltage below a predetermined maximum voltage threshold.

In another aspect, a transition of the motor speed from the inactive state to the operating speed occurs over a startup time duration of less than 8 seconds.

In another aspect, the method further includes displacing an impeller of the blood pump from a starting position in which the impeller is in contact with a disk to an operating position in which the impeller is displaced away from the disk.

In another aspect, the method further includes generating a non-linear frequency ramp when gradually increasing the motor speed from the inactive state to the first speed.

In another aspect, the method further includes applying a predetermined maximum voltage to a plurality of coils of a stator disposed within the implantable blood pump when gradually increasing the motor speed from the inactive state to the first speed.

In another aspect, the inactive state includes a speed of zero and the first speed being between 100 to 2000 RPM.

In another aspect, the first-time period is between 2 to 3 seconds and the second-time period is less than one second.

In one aspect, a method of starting an implantable blood pump including a first stator and a second stator includes detecting an electrical fault in one of a group consisting of the first stator and the second stator. A startup of the first stator and the second stator is commenced in response to the electrical fault.

In another aspect, the method further includes operating the first stator in at least three phases and the second stator in a set of two phases when detecting the electrical fault in the second stator.

In another aspect, the method further includes using the first stator for measuring back electromotive force.

In another aspect, the first stator and the second stator each include a set of three motor windings.

In another aspect, the method further includes increasing an axial force and a radial torque relative to a predetermined amount to displace an impeller from a disk within the implantable blood pump.

In another aspect, the method further includes alternating a phase angle of the first stator.

In one aspect, a method of starting an implantable blood pump includes performing a plurality of motor startup attempts including applying a first voltage less than a predetermined maximum voltage to a plurality of stators. In response to a failure of the plurality of motor startup attempts, an additional startup attempt is performed including applying the predetermined maximum voltage to the plurality of stators.

In another aspect, the plurality of motor startup attempts includes a set of two startup attempts.

In another aspect, the method further includes detecting whether a motor startup has occurred after each of the plurality of motor startup attempts.

In another aspect, the method further includes gradually increasing a motor speed from an inactive state to an operating speed.

In another aspect, the predetermined maximum voltage is between 10-14V.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 depicts an exploded view of an exemplary implantable blood pump;
FIG. 2 depicts an example control circuit configured to monitor and control startup of a motor of the implantable blood pump of FIG. 1;
FIG. 3 is a flow chart illustrating an exemplary startup routine for controlling startup of the motor of FIG. 2;
FIG. 4 is a graph depicting one or more pump parameters associated with the startup routine of FIG. 3;
FIG. 5 is a flow chart illustrating another exemplary startup routine for controlling startup of the motor of FIG. 2;
FIG. 6 is a schematic of an exemplary power supply, pump controller, impeller, and a pair of stators of the implantable blood pump of FIG. 1;
FIG. 7 is a flow chart illustrating another exemplary startup routine for controlling startup of the motor of FIG. 2; and
FIG. 8 is a graph depicting one or more pump parameters associated with the startup routine of FIG. 7.

### DETAILED DESCRIPTION

Before describing in detail exemplary embodiments, it is noted that the embodiments reside primarily in combinations of apparatus components and processing steps related to starting a blood pump. Accordingly, the system and method components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein.

As used herein, relational terms, such as "first" and "second," "top" and "bottom," and the like, may be used solely to distinguish one entity or element from another entity or element without necessarily requiring or implying any physical or logical relationship or order between such entities or elements. The terminology used herein is for the purpose of describing particular embodiments only and is not intended to be limiting of the concepts described herein. As used herein, the singular forms "a", "an" and "the" are intended to include the plural forms as well, unless the context clearly indicates otherwise. It will be further understood that the terms "comprises," "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms used herein should be interpreted as having a meaning that is consistent with their meaning in the context of this specification and the relevant art and will not be interpreted in an idealized or overly formal sense unless expressly so defined herein.

In embodiments described herein, the joining term, "in communication with" and the like, may be used to indicate electrical or data communication, which may be accomplished by physical contact, induction, electromagnetic radiation, radio signaling, infrared signaling or optical signaling, for example. One having ordinary skill in the art will appreciate that multiple components may interoperate and modifications and variations are possible of achieving the electrical and data communication.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

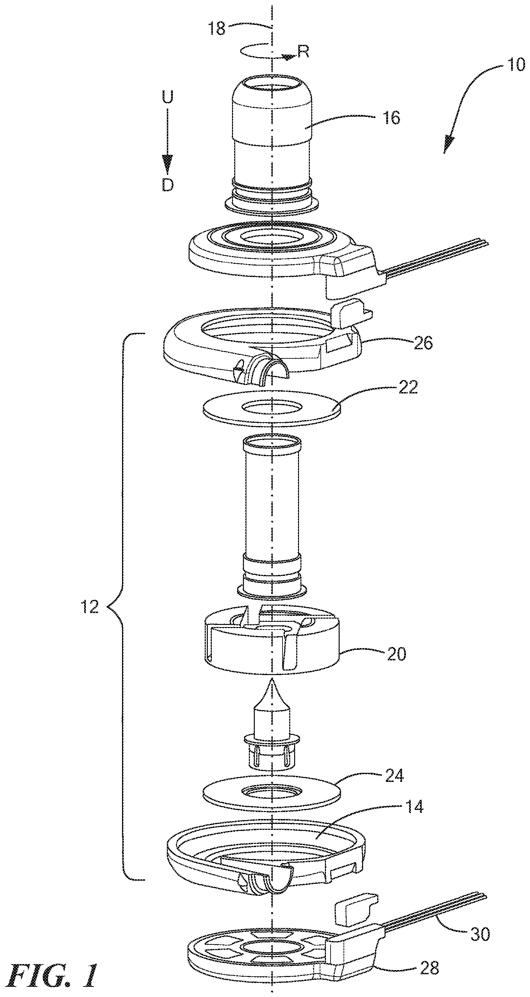

Referring now to the drawings in which like reference designators refer to like elements there is shown in FIG. 1 an exemplary blood pump constructed in accordance with the principles of the present application and designated generally "10." The general arrangement of the blood pump components may be the same or similar to the HVAD^{®} Pump described in U.S. Patent Nos. 7,997,854 and 8,512,013, the disclosures of which are incorporated by reference herein in the entirety. For example, the blood pump 10 may include a housing 12 having a chamber 14, an inflow cannula 16, and a major longitudinal axis 18 extending therethrough. An enclosed flow path extends along the axis 18 from an upstream to a downstream direction, as indicated by the arrows U and D, respectively. A generally disc-shaped ferromagnetic impeller 20 is mounted within the chamber 14 between a first ceramic disk 22 and a second ceramic disk 24 for rotation about the axis 18.

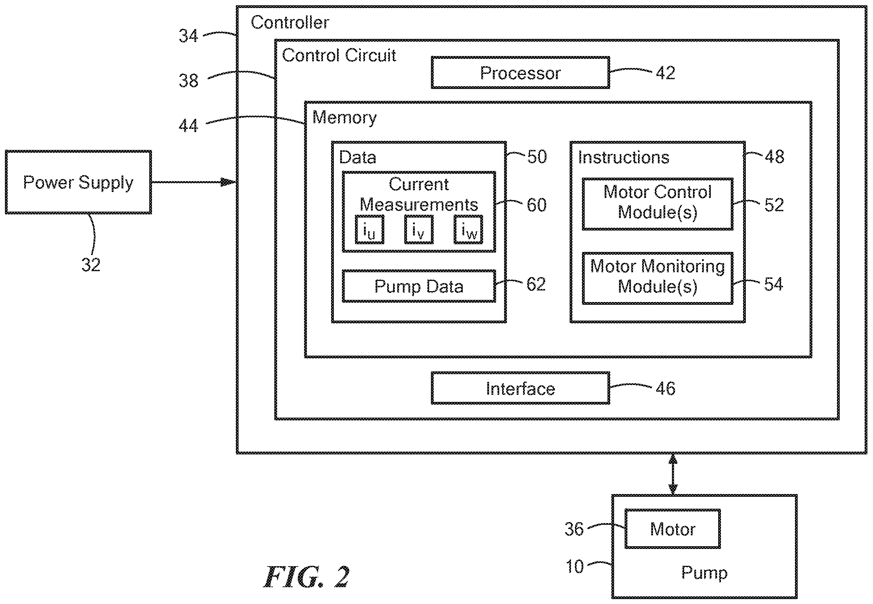

The blood pump 10 may be arranged so that the impeller 20 is levitated within the housing 12 by contactless bearings, such as magnetic bearings, hydrodynamic bearings or a combination of the two. For example, the blood pump 10 may include a first stator 26 and a second stator 28 disposed within the housing 12. The first stator 26 may be located proximate the first ceramic disk 22 and the second stator 28 may be located proximate the second ceramic disk 24. In operation, a voltage may be applied to one or more coils of the first stator 26 and/or the second stator 28 to rotate the impeller 20 to impel the blood. An electrical connector 30 may supply the voltage to the coils from, as shown in FIG. 2, a power supply 32 such as an external AC power supply, external battery, implanted battery, or any combination thereof, coupled to or stored within a controller 34.

With reference to FIGS. 1 and 2, the first stator 26 and the second stator 28 may operate in combination or independent of each other and may each form a portion of a sensorless three-phase brushless direct-current ("BLDC") motor 36. In one configuration, the coils of the first stator 26 and the second stator 28 are in the form of three motor windings controlled by a different respective phase U, V, W, of a power input for three-phase motor control. The BLDC motor includes an inverter circuit to convert a DC input to the three-phase output. Alternatively, the blood pump 10 may receive an alternating current (AC) three-phase input. Examples of three-phase motor control methods and devices are provided in commonly owned and co-pending U.S. Application Nos. 62/271,278, and 15/710323, the disclosures of which are incorporated herein in the entirety.

FIG. 2 shows an example control circuit 38 coupled to the blood pump 10 including hardware and software for monitoring and controlling startup and subsequent operation of one or both of the stators 26 and 28 according to the routines of the present disclosure. The control circuit 38 includes a processor 42, a memory 44, and an interface 46 for interfacing with the motor 36. The memory 44 stores information accessible by the processor 42, including instructions 48 that may be executed by the processor 42. The memory 44 also includes data 50 that may be retrieved, manipulated or stored by the processor 42. Further details associated with the control circuit 38 are provided in commonly owned and co-pending U.S. Application No. 15/710323, the disclosure of which is incorporated herein in the entirety.

The instructions 48 stored in the memory 44 may include one or more instruction sets or modules, for performing certain operations in accordance with the present disclosure. One such module may be a motor control module 52 for controlling operation of the motor 36 (e.g., increasing or decreasing current supplied to the motor). The instructions may also include one or more motor monitor modules 54 for monitoring operation of the motor 36. Examples of motor control and monitoring modules may be found in any of the commonly owned and co-pending U.S. Application Serial Nos. 13/355,297, 13/951,302, 14/294,448, 14/950,467, 62/266,871, and 62/271,618, the disclosures of which are hereby incorporated herein by reference in their entireties.

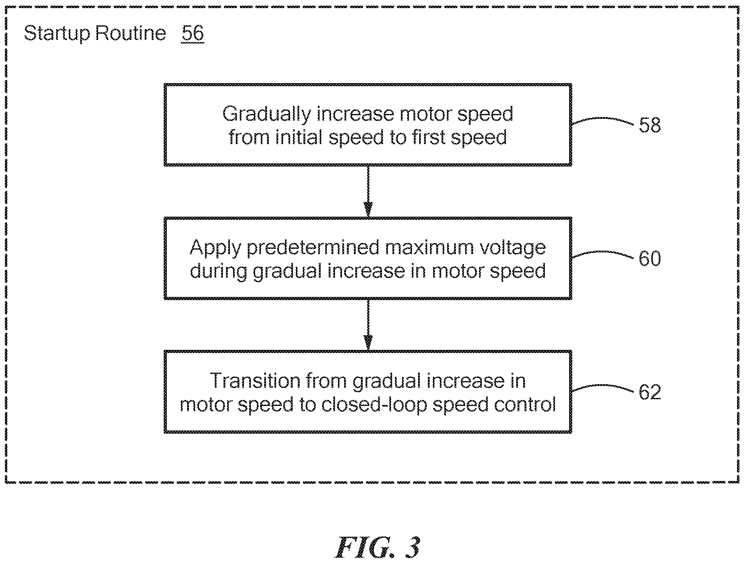

Referring now to FIG. 3, a startup routine 56 may be executed by the control circuit 38 of FIG. 2, or by a similarly capable control circuit coupled to the controller 34. The startup routine 56 generates a relatively higher amount of initial torque than that which may be produced during one or more alternative startup routines. As such, the startup routine 56 may be activated after a failure of another startup routine and thus may be referred to as a secondary startup routine. The startup routines disclosed herein are configured to displace the impeller 20 from a starting position at rest, in which the impeller 20 is in contact with the first disk 22 and/or the second disk 24, to an operating position in which the impeller 20 is suspended within the housing 12 to impel the blood.

In one configuration, the startup routine 56 begins at step 58 with the control circuit 38 instructing the motor 36 to gradually increase a motor speed during a ramp phase from an inactive state, including a speed of zero when the impeller 20 is at rest, to an operating speed. During the speed increase, at step 60, the control circuit 38 applies a predetermined maximum voltage, creating high torque to overcome friction or coagulated blood that may hinder the movement of the impeller 20. At step 62, the control circuit 38 transitions or switches from the gradual increase in motor speed to a closed-loop speed control to achieve the operating speed in which the blood pump operates in a normal mode. The closed-loop speed control may be performed by the control circuit 38 of the controller (FIG. 2). For example, the motor speed may be maintained by adjusting a voltage according to a speed difference produced by the torque.

In response to the predetermined maximum voltage still applied, the transition to closed-loop control may cause an overshoot in the motor speed in which the motor speed increases from a first speed to an overshoot speed greater than the operating speed. In other words, rather than a control loop increasing from the inactive state directly to the operating speed, an overshoot of the operating speed may inadvertently occur. This overshoot may be minimized by appropriate tuning of the closed-loop speed-control parameters.

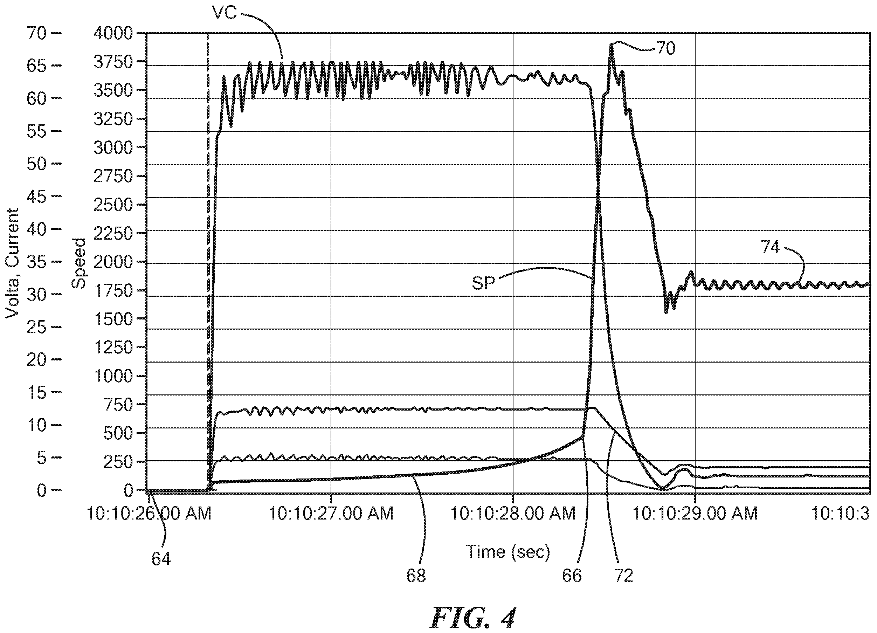

With reference to FIG. 4, a speed waveform "SP" is depicted in a solid line illustrating the gradual increase in motor speed from an initial speed 64 of zero in the inactive state to the first speed 66. The first speed 66 may be between 250 to 600 RPM, such as 480 RPM, or as otherwise preprogrammed in accordance with the pump parameters. The term "gradual" includes the speed change occurring within a first-time period 68, such as 2 to 3 seconds. During the first-time period 68, the speed waveform is produced as a non-linear frequency ramp. The control circuit 38 may include a timer or timing mechanism configured to control the changes in timing of the motor speed.

In one configuration, the predetermined maximum voltage is applied during the ramp phase of the startup routine 56. The predetermined maximum voltage has a corresponding torque output that is relatively high in comparison to the amount of torque existing in other phases of the motor startup. For example, as depicted using a voltage/current waveform "VC" represented in a solid line, the voltage is increased from zero to the predetermined maximum voltage during the first-time period 68 beginning with initial activation. The predetermined maximum voltage may be between 10 to 14V and the first-time period 68 may be under one second. The controller 34 may be programmed to maintain the voltage below a predetermined maximum voltage threshold, such as 14V, or as otherwise designated. The increase in the motor speed may result in a speed phase that directly follows the ramp phase, thereby bypassing a commutation phase. In other words, the startup routine 56 is executed without incrementally ramping the motor voltage and searching for or measuring back electromotive force, i.e., "BEMF" or "Back-EMF," signals indicating impeller motion or positioning, as is commonly found during the commutation phase. The speed phase may include the overshoot and may occur during a second-time period 72 less than the first-time period 68, such as less than one second, for example between 0.25 to 0.75 seconds. In other words, the motor speed increases at a faster rate in the speed phase when compared to the speed increase in the ramp phase. Ordinary operation of the motor 36 may commence when the motor 36 reaches the operating speed 74, such as between 1800 to 4000 RPM. Overall, the ramp and speed phases, including the motor speed transition from the initial speed of zero to the operating speed 74, occur over a startup time duration that is less than 8 seconds, such as between 3-4 seconds.

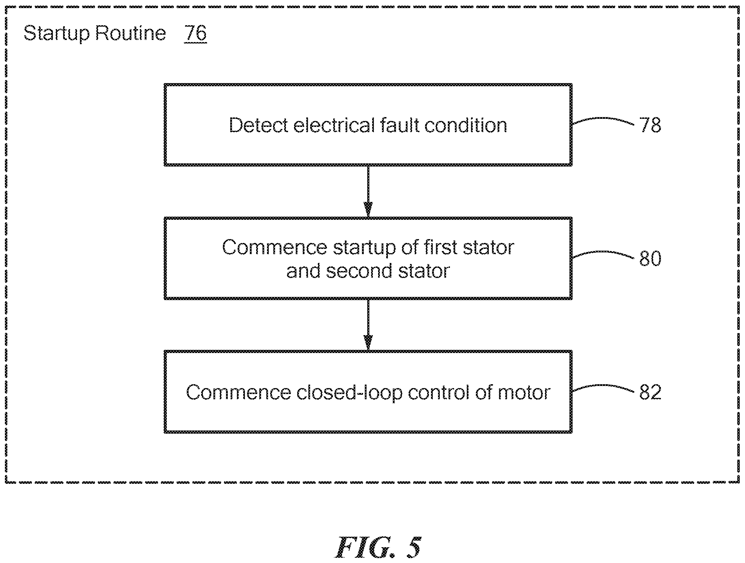

With reference to FIG. 5, the control circuit 38 or a similarly capable control circuit coupled to the controller 34, performs a startup routine 76 including detecting an electrical fault in the first stator 26 or the second stator 28. The startup routine 76 may be referred to as a 5-wire start. For example, in one configuration, the startup routine 76 begins at step 78 with detecting the electrical fault, for example, in the second stator 28, such as using the control circuit 38. At step 80, startup of the first stator 26 and the second stator 28 are commenced, such as by applying a voltage thereto from the power supply, in response to the electrical fault condition. In other words, despite the presence of the electrical fault condition, both stators are started. At step 82, closed-loop control of the motor may be commenced including the non-faulty stator being used for monitoring BEMF. In another configuration, the electrical fault may be detected after startup is completed, such as when the stators are running at the operating speed in the normal operative mode. Detection of the electrical fault in the second stator 28 is provided for illustrative purposes only as the electrical fault condition may exist in either stator.

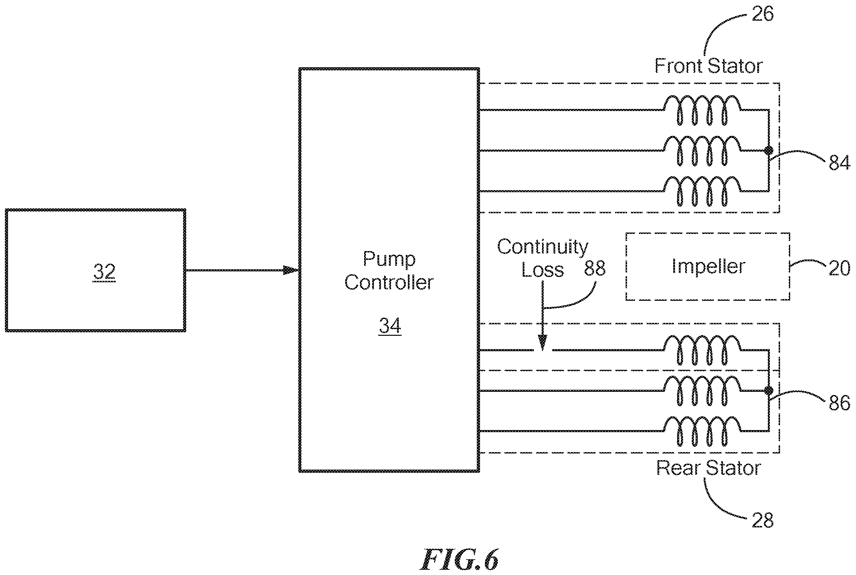

With reference to FIG. 6, further details associated with the startup routine 76 are depicted including the power supply 32 and the controller 34 being in communication with the first stator 26 having the three motor windings 84 and the second stator 28 having the three motor windings 86 on opposing sides of the impeller 20. The controller 34 commences startup of the first stator 26 by driving the three motor windings 84 using voltage and current from the power supply 32 such that the first stator 26 operates in the three phases, e.g., U, V, W. Commencing startup of the first stator 26 generates a predetermined amount of axial force and radial torque for rotating and suspending the impeller 20.

Before the first stator 26 and the second stator 28 are started, as mentioned above, the controller 34 may detect the electrical fault condition, such as a continuity loss 88 in one or more of the motor windings 86 of the second stator 28. Despite the electrical fault condition, the controller 34 may commence the startup of the second stator 28 such that the second stator 28 operates in a set of two phases, thereby providing a five-wire start between the combined stators 26, 28. Commencing startup of the second stator 28, even in the presence of the electrical fault condition, generates a greater amount of axial force and radial torque than that which is generated using only a single stator to increase the likelihood of a successful startup. The successful startup includes the impeller 20 being rotated and suspended within the housing 12 to impel the blood in a normal operative mode. In situations in which the electrical fault is detected in either stator after startup is completed, the first stator 26 and the second stator 28 may be configured to continue operating in the normal operative mode at the operating speed despite the presence of the electrical fault condition.

The two operating phases of the second stator 28, or faulty stator, may vary in accordance with the location of the continuity loss 88 with respect to the coils in an effort to rotate and suspend the impeller 20. In other words, the phase angle of the first stator 26 and/or the second stator 28 with respect to the housing 12 may be altered depending upon the location of the faulty stator within the housing 12, e.g., proximate the first disk 22 or the second disk 24, and the location of the electrical fault condition. For example, the control circuit 38 may alter the wire phase of the first stator 26 and/or the second stator 28 to alter the phase angle and vary the torque in an effort to rotate the impeller 20.

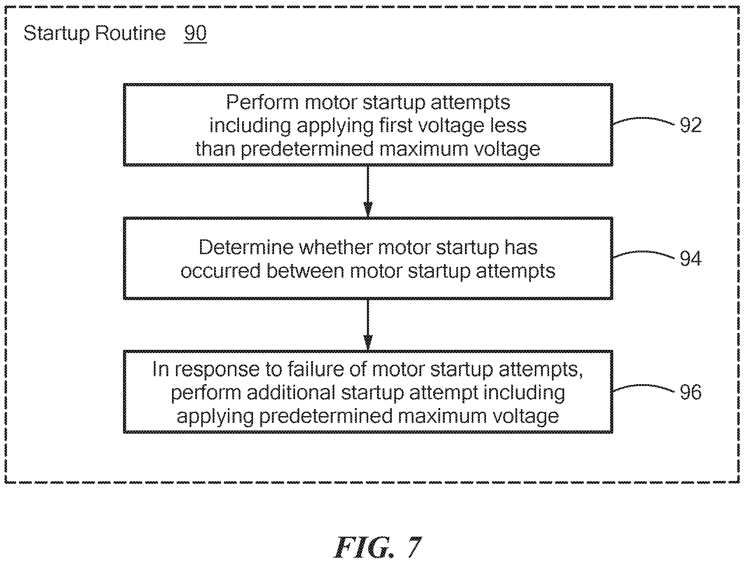

Referring now to FIG. 7, the control circuit 38 or a similarly capable control circuit coupled to the controller 34, performs a startup routine 90 in which a first voltage is applied to the stators 26, 28, that is less than a predetermined maximum voltage. In one configuration, the predetermined maximum voltage is the predetermined maximum voltage that is below the predetermined maximum voltage threshold discussed above with respect to the startup routine 56. As such, the startup routine 90 attempts the motor startup using an amount of torque less than the amount of torque initially generated during the startup routine 56 in an attempt to decrease the risk of harm to the patient that may occur with the higher amount of torque. Accordingly, the startup routine 90 may be referred to as a primary startup routine with the startup routine 56 being the secondary startup routine.

In one configuration, the startup routine 90 begins at step 92 with a first motor startup attempt including applying a first voltage to both stators 26, 28, such as from the power supply 32. If unsuccessful, the control circuit 38 executes a second startup attempt in the manner described with respect to the first startup attempt. In one configuration, two startup attempts are performed with an interval of 2 to 3 seconds therebetween; however, other configurations may include a single startup attempt and/or more than two startup attempts with a shorter or longer interval therebetween.

At step 94, after each motor startup attempt, the control circuit 38 detects whether the motor 36 was successfully started. In response to a failure of the motor startup attempts, at step 96, the control circuit 38 performs an additional startup attempt using the startup routine 56. In other words, the additional startup attempt includes applying the predetermined maximum voltage, which is greater than the first voltage, to the stators 26, 28, and implementing the speed ramp of the startup routine 56.

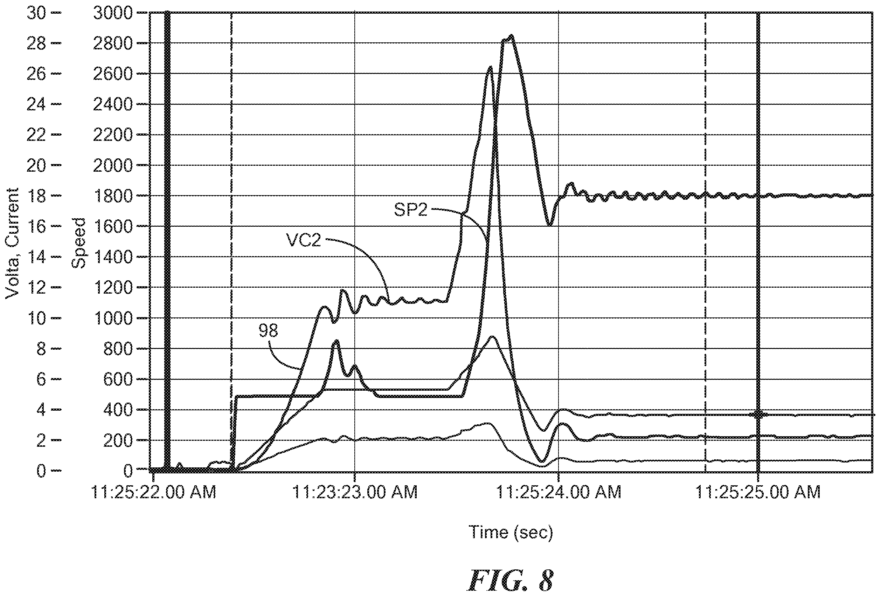

For example, with reference to FIG. 8, the first and second motor startup attempts include applying the first voltage, depicted using the voltage current waveform "VC2," to the first stator 26 and/or the second stator 28 during a first-time period. In one configuration, the first voltage is between 8 to 12V and the first-time period includes the first stator 26 and the second stator 28 being in the inactive state. The first voltage and the predetermined maximum voltage may be stored in the memory of the controller 34.

If the startup attempt is successful, during the ramp phase, the first voltage may increase from zero to a range below the predetermined maximum voltage threshold in less than one second. In addition, the motor speed, depicted using a speed waveform "SP2" shown in a solid line, will undergo stepwise changes during the ramp phase, a commutation phase, and a speed phase that may exceed a startup duration of more than 10 seconds. Whether or not the startup is successful may be determined through one or more methods, such as the control circuit 38 attempting to search for and/or measure the BEMF signals indicating the motion or positioning of the impeller 20

If the startup attempts, such as the two startup attempts, are not successful in starting the motor 36 and displacing the impeller 20 from the disks 22, 24, the additional startup attempt is performed. In one configuration, the additional startup attempt is the startup routine 56 which includes applying the predetermined maximum voltage, such as 14V, to the first stator 26 and/or the second stator 28 from the inactive state and transitioning the motor speed from the ramp phase directly to the speed phase to the exclusion of the commutation phase. In other configurations, an alternative amount of voltage may be used during another type of startup routine.

Certain embodiments of the invention include:
Embodiment 1. A method of starting an implantable blood pump comprising:
   gradually increasing a motor speed from an inactive state to a first speed during a first- time period;
   applying a predetermined maximum voltage during the gradual increase in the motor speed; and
   transitioning from the gradual increase in motor speed to a closed loop speed control to achieve an operating speed, the operating speed being greater than the first speed.
Embodiment 2. The method of Embodiment 1, further comprising bypassing a commutation phase.
Embodiment 3. The method of Embodiment 1, further comprising maintaining a voltage below a predetermined maximum voltage threshold.
Embodiment 4. The method of Embodiment 3, wherein a transition of the motor speed from the inactive state to the operating speed occurs over a startup time duration of less than 8 seconds.
Embodiment 5. The method of Embodiment 4, further comprising displacing an impeller of the blood pump from a starting position in which the impeller is in contact with a disk to an operating position in which the impeller is displaced away from the disk.
Embodiment 6. The method of Embodiment 1, further comprising generating a non-linear frequency ramp when gradually increasing the motor speed from the inactive state to the first speed.
Embodiment 7. The method of Embodiment 1, further comprising applying a predetermined maximum voltage to a plurality of coils of a stator disposed within the implantable blood pump when gradually increasing the motor speed from the inactive state to the first speed.
Embodiment 8. The method of Embodiment 1, wherein the inactive state includes a speed of zero and the first speed being between 100 to 2000 RPM.
Embodiment 9. The method of Embodiment 1, wherein the first-time period is between 2 to 3 seconds and the second-time period is less than one second.
Embodiment 10. A method of starting an implantable blood pump including a first stator and a second stator, the method comprising:
   detecting an electrical fault in one of a group consisting of the first stator and the second stator; and
   commencing a startup of the first stator and the second stator in response to the electrical fault.
Embodiment 11. The method of Embodiment 10, further comprising operating the first stator in at least three phases and the second stator in a set of two phases when detecting the electrical fault in the second stator.
Embodiment 12. The method of Embodiment 11, further comprising using the first stator for measuring back electromotive force.
Embodiment 13. The method of Embodiment 10, wherein the first stator and the second stator each include a set of three motor windings.
Embodiment 14. The method of Embodiment 10, further comprising increasing an axial force and a radial torque relative to a predetermined amount to displace an impeller from a disk within the implantable blood pump.
Embodiment 15. The method of Embodiment 10, further comprising alternating a phase angle of the first stator.
Embodiment 16. A method of starting an implantable blood pump comprising: performing a plurality of motor startup attempts including applying a first voltage less than a predetermined maximum voltage to a plurality of stators; and in response to a failure of the plurality of motor startup attempts, performing an additional startup attempt including applying the predetermined maximum voltage to the plurality of stators.
Embodiment 17. The method of Embodiment 16, wherein the plurality of motor startup attempts includes a set of two startup attempts.
Embodiment 18. The method of Embodiment 16, further comprising detecting whether a motor startup has occurred after each of the plurality of motor startup attempts.
Embodiment 19. The method of Embodiment 16, further comprising gradually increasing a motor speed from an inactive state to an operating speed.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope and spirit of the invention, which is limited only by the following claims.

Further disclosed herein is the subject matter of the folliwing clauses:
1. A control circuit for an implantable blood pump, the control circuit having processing circuity including one or more processors, the one or more processors being configured to:
   gradually increase a motor speed from an inactive state to a first speed during a first- time period;
   apply a predetermined maximum voltage during the gradual increase in the motor speed; and
   transition from the gradual increase in motor speed to a closed loop speed control to achieve an operating speed, the operating speed being greater than the first speed.
2. The control circuit of clause 1, wherein the one or more processors are further configured to bypass a commutation phase.
3. The control circuit of clause 1, wherein the one or more processors are further configured to maintain a voltage below a predetermined maximum voltage threshold.
4. The control circuit of clause 3, wherein a transition of the motor speed from the inactive state to the operating speed occurs over a startup time duration of less than 8 seconds.
5. The control circuit of clause 4, wherein the one or more processors are further configured to, displace an impeller of the blood pump from a starting position in which the impeller is in contact with a disk to an operating position in which the impeller is displaced away from the disk.
6. The control circuit of clause 1, wherein the one or more processors are further configured to generate a non-linear frequency ramp when gradually increasing the motor speed from the inactive state to the first speed.
7. "The control circuit of clause 1, wherein the one or more processors are further configured to apply a predetermined maximum voltage to a plurality of coils of a stator disposed within the implantable blood pump when gradually increasing the motor speed from the inactive state to the first speed.
8. The control circuit of clause 1, wherein the inactive state includes a speed of zero and the first speed being between 100 to 2000 RPM.
9. The control circuit of clause 1, wherein the first-time period is between 2 to 3 seconds and the second-time period is less than one second.
10. A control circuit for an implantable blood pump, the blood pump having a first stator and a second stator, the control circuit having processing circuity including one or more processors, the one or more processors being configured to:
   detectan electrical fault in one of a group consisting of the first stator and the second stator; and
   commence a startup of the first stator and the second stator in response to the electrical fault.
11. The control circuit of clause 10, wherein the one or more processors are further configured to operate the first stator in at least three phases and the second stator in a set of two phases when detecting the electrical fault in the second stator.
12. The control circuit of clause 11, wherein the one or more processors are further configured to use the first stator for measuring back electromotive force.
13. The control circuit of clause 10, wherein the first stator and the second stator each include a set of three motor windings.
14. The control circuit of clause 10, wherein the one or more processors are further configured to increase an axial force and a radial torque relative to a predetermined amount to displace an impeller from a disk within the implantable blood pump.
15. The control circuit of clause 10, wherein the one or more processors are further configured to alternate a phase angle of the first stator.

## Claims

1. A control circuit for an implantable blood pump, the blood pump having a first stator and a second stator, the control circuit having processing circuity including one or more processors, the one or more processors being configured to:
detect an electrical fault in one of a group consisting of the first stator and the second stator; and
commence a startup of the first stator and the second stator in response to the electrical fault.

2. The control circuit of Claim 1, wherein the one or more processors are further configured to operate the first stator in at least three phases and the second stator in a set of two phases when detecting the electrical fault in the second stator.

3. The control circuit of any preceding Claim, wherein the one or more processors are further configured to use the first stator for measuring back electromotive force.

4. The control circuit of any preceding Claim, wherein the first stator and the second stator each include a set of three motor windings.

5. The control circuit of any preceding Claim, wherein the one or more processors are further configured to increase an axial force and a radial torque relative to a predetermined amount to displace an impeller from a disk within the implantable blood pump.

6. The control circuit of any preceding Claim, wherein the one or more processors are further configured to alternate a phase angle of the first stator.

7. A method of starting an implantable blood pump including a first stator and a second stator, the method comprising:
detecting an electrical fault in one of a group consisting of the first stator and the second stator; and
commencing a startup of the first stator and the second stator in response to the electrical fault.

8. The method of claim 7, further comprising operating the first stator in at least three phases and the second stator in a set of two phases when detecting the electrical fault in the second stator.

9. The method of claim 7 or 8, further comprising using the first stator for measuring back electromotive force.

10. The method of any one of claims 7 to 9, wherein the first stator and the second stator each include a set of three motor windings.

11. The method of any one of claims 7 to 10, further comprising increasing an axial force and a radial torque relative to a predetermined amount to displace an impeller from a disk within the implantable blood pump.

12. The method of any one of claims 7 to 11, further comprising alternating a phase angle of the first stator.
